# FASCICULE DE BREVET EUROPEEN

(11) **EP 1 222 181 B1**
(45) Date de publication et mention de la délivrance du brevet: **10.12.2003**
(21) Numéro de dépôt: 00971484.1
(22) Date de dépôt: 23.10.2000
(51) Int. Cl.: C07D 307/79, C07D 333/54, C07B 37/10

(54) **PROCEDE DE PREPARATION D'UN COMPOSE DE TYPE BENZOFURANE OU BENZOTHIOPHENE**
VERFAHREN ZUR HERSTELLUNG VON BENZOFURANEN ODER BENZOTHIOPHENEN
METHOD FOR PREPARING A BENZOFURAN OR BENZOTHIOPHENE COMPOUND

(30) Priorité: 21.10.1999 FR 9913251; 24.05.2000 FR 0006629
(43) Date de publication de la demande: 17.07.2002
(73) Titulaire: RHODIA CHIMIE, 92408 Courbevoie Cédex (FR)
(72) Inventeur: SCHLAMA, Thierry, F-69570 Dardilly (FR)
(74) Mandataire: Dutruc-Rosset, Marie-Claude
(86) Numéro de dépôt international: FR0002947
(87) Numéro de publication internationale: WO01029019

(56) Documents cités:
- EP-A- 0 471 609
- US-A- 3 577 441
- T. SUZUKI: "Benzofuran derivatives. I. On the effect of substituents in benzofuran synthesis" BULLETIN OF THE CHEMICAL SOCIETY OF JAPAN, vol. 56, no. 9, septembre 1983 (1983-09), pages 2762-2767, XP002141555 TOKYO JP
- W. T. BRADY: "Benzofurans. Ketene intermediates in the Perkin reaction" JOURNAL OF HETEROCYCLIC CHEMISTRY, vol. 25, 1988, pages 969-971, XP002141556

## Description

La présente invention a pour objet un nouveau procédé de préparation d'un composé de type benzofurane ou benzothiophène par cyclisation d'un composé aromatique comprenant un cycle benzénique porteur d'une part, d'une chaîne latérale comprenant au moins deux atomes de carbone, l'un des atomes de carbone étant relié au cycle benzénique par un atome d'oxygène ou de soufre, l'autre atome de carbone est sous forme carboxylique et d'autre part d'un groupe formyle en position ortho par rapport à ladite chaîne.

L'invention vise plus particulièrement la préparation du 2-n-butyl-5-nitrobenzofurane.

Des structures de type benzofurane ou benzothiophène se rencontrent dans de nombreuses molécules utilisées dans le domaine pharmaceutique. En particulier, on a décrit dans EP-A-0 471 609, un procédé de préparation de 2-n-butyl-5-nitro-benzofurane qui consiste à faire réagir le bromure de 2-hydroxy-5-nitro-benzyltriphénylphosphonium avec le chlorure de pentanoyle en présence de pyridine : le bromure de 2-hydroxy-5-nitro-benzyltriphénylphosphonium étant obtenu à partir du bromure de 2-hydroxy-5-nitrobenzyle et de triphénylphosphine
L'objectif de la présente invention est de fournir un autre procédé de préparation en phase liquide permettant d'obtenir le 5-nitrobenzofurane avec un très bon rendement réactionnel.

Il a maintenant été trouvé et c'est ce qui constitue l'objet de la présente invention un procédé de préparation d'un composé de type benzofurane ou benzothiophène à partir d'un composé aromatique comprenant un cycle benzénique porteur d'une part, d'une chaîne latérale comprenant au moins deux atomes de carbone, l'un des atomes de carbone étant relié au cycle benzénique par un atome d'oxygène ou de soufre, l'autre atome de carbone est sous forme carboxylique et d'autre part d'un groupe formyle en position ortho par rapport à ladite chaîne. caractérisé par le fait qu'il consiste à effectuer la cyclisation de ce dernier, dans un milieu comprenant un anhydride d'acide carboxylique et en présence d'une base choisie parmi les carbonates et/ou hydrogénocarbonates métalliques ou d'ammonium.

Une variante préférée du procédé de l'invention consiste à préparer le composé de type benzofurane ou benzothiophène à partir d'un composé aromatique comprenant un cycle benzénique porteur d'une part, d'une chaîne latérale comprenant au moins deux atomes de carbone, l'un des atomes de carbone étant relié au cycle benzénique par un atome d'oxygène ou de soufre, l'autre atome de carbone est sous forme carboxylique et d'autre part d'un groupe formyle en position ortho par rapport à ladite chaîne en effectuant la cyclisation de ce dernier, en présence d'une quantité efficace d'une base telle que précitée, dans un milieu comprenant un anhydride d'acide carboxylique et en présence d'un solvant organique.

On a trouvé qu'en mettant en oeuvre un solvant organique tel que par exempte le diméthylformamide, on facilite la récupération du produit cyclisé tout en permettant de diminuer la quantité d'anhydride d'acide carboxylique mise en oeuvre.

Le réactif de départ du procédé de l'invention est un composé aromatique de préférence benzénique qui porte une chaîne latérale et un groupe formyle en position ortho de la chaîne latérale.

La chaîne latérale présente 3 caractéristiques :
- un atome d'oxygène ou de soufre relie la chaîne latérale au cycle benzénique,
- un nombre d'atomes de carbone de 2 enchaînés linéairement intervenant dans le cycle à 5 atomes qui fait également intervenir les 2 atomes de carbone du cycle benzénique,
- un groupe carboxylique ou thiocarboxylique en position β par rapport à l'atome d'oxygène ou de soufre.

Ainsi, le réactif de départ peut être symbolisé par la forme suivante : 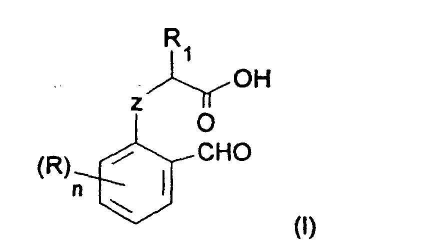 dans ladite formule (I) :
- R₁ représente un atome d'hydrogène, un groupe alkyle linéaire ou ramifié ayant de 1 à 12 atomes de carbone, un groupe phényle éventuellement substitué par un groupe alkyle ayant de 1 à 4 atomes de carbone ou un groupe halogénophényle,
- Z représente un atome d'oxygène ou de soufre,
- R représente un atome d'hydrogène ou un substituant,
- n est un nombre égal à 0,1, 2, ou 3, de préférence, égal à 0,
- lorsque n est supérieur à 1, deux groupes R et les 2 atomes successifs du cycle benzénique peuvent former entre eux un cycle saturé, insaturé ou aromatique ayant de 5 à 7 atomes de carbone.

Dans la formule (I), le cycle benzénique peut porter un substituant.

L'invention n'exclut pas la présence sur le cycle benzénique de tout type de substituant dans la mesure où il ne réagisse pas dans les conditions de l'invention.

Comme exemples plus particuliers de groupes R, on peut mentionner entre autres :
- un groupe nitro,
- un groupe hydroxyle,
- un groupe alkyle linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
- un groupe alkoxy ayant de 1 à 6 atomes de carbone, de préférence de 1 à 4 atomes de carbone,
- un groupe ester ayant de 1 à 10 atomes de carbone, de préférence de 1 à 4 atomes de carbone,
- un groupe alkylamide ayant de 1 à 6 atomes de carbone, de préférence 1 à 4 atomes de carbone,
- un groupe carboxamide,
- un atome d'halogène,
- un groupe trifluorométhyle.

Lorsque n est supérieur à 1, deux groupes R et les 2 atomes successifs du cycle benzénique peuvent former entre eux un cycle saturé, insaturé ou aromatique ayant de 5 à 7 atomes de carbone, de préférence 6 atomes de carbone. Ainsi, deux groupes R forment avantageusement un cycle benzénique.

Le groupe R₁ est avantageusement un groupe alkyle ayant de 1 à 4 atomes de carbone.

Les substrats préférés du procédé de l'invention répondent à la formule (I) dans laquelle R représente un atome d'hydrogène, un groupe nitro, un groupe méthyle ou éthyle, un groupe méthoxy ou éthoxy.

Dans la formule (I), Z représente préférentiellement un atome d'oxygène.

Dans la formule (I), R peut représenter un groupe nitro de préférence en position méta par rapport au groupe formyle.

Conformément au procédé de l'invention, on effectue la cyclisation du composé aromatique répondant de préférence, à la formule (I), en présence d'une base qui est choisie parmi les carbonates et/ou hydrogénocarbonates métalliques ou d'ammonium.

Comme bases susceptibles d'être utilisées, on peut mentionner tout particulièrement les carbonates et les hydrogénocarbonates de métaux alcalins ou alcalino-terreux. On peut faire appel au carbonate de césium mais l'on préfère le carbonate de sodium ou le carbonate de potassium.

Conformément au procédé de l'invention, on effectue la cyclisation du composé aromatique répondant de préférence, à la formule (I), dans un anhydride d'acide carboxylique.

Ce dernier répond plus particulièrement à la formule suivante : 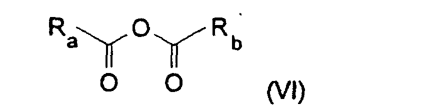 dans ladite formule (VI) :
- Rₐ et R_{b}, identiques ou différents, représentent un groupe hydrocarboné monovalent, substitué ou non qui peut être un groupe aliphatique acyclique, saturé ou insaturé, linéaire ou ramifié ; un groupe carbocyclique saturé, insaturé ou aromatique, monocyclique,
- Rₐ et R_{b} pouvant former ensemble un groupe divalent aliphatique saturé ou insaturé, linéaire ou ramifié, ayant au moins 2 atomes de carbone.

Les groupes Rₐ et R_{b} sont choisis préférentiellement de telle sorte que l'anhydride soit liquide dans les conditions réactionnelles.

L'anhydride mis en oeuvre peut être cyclique ou non.

Plus précisément, on fait appel à un anhydride cyclique ayant de 5 à 10 atomes dans le cycle ne comprenant pas ou comprenant une double liaison, l'un des atomes pouvant être remplacé par un atome d'oxygène.

Les anhydrides cycliques mis en oeuvre préférentiellement sont saturés ou comprennent une double liaison et possèdent 5 ou 6 atomes dans le cycle.

Le cycle peut comprendre un ou plusieurs substituants. Comme exemples plus particuliers de substituants, on peut citer, notamment les groupes alkyle, linéaires ou ramifiés, ayant de 1 à 12 atomes de carbone, de préférence de 1 à 4 atomes de carbone, tel que méthyle, éthyle, propyle, isopropyle, butyle, isobutyle, sec-butyle, tert-butyle ou des atomes d'halogène ou un groupe trihalogénométhyle.

Pour ce qui de la mise en oeuvre d'un anhydride non cyclique répondant à la formule (VI), les groupes Rₐ et R_{b}, identiques ou différents représentent plus particulièrement :
- un groupe aliphatique acyclique linéaire ou ramifié ayant de préférence de 1 à 24, de préférence, de 1 à 12 atomes de carbone, saturé ou comprenant une à plusieurs insaturations sur la chaîne, généralement, 1 à 3 insaturations qui peuvent être des doubles liaisons simples : la chaîne hydrocarbonée pouvant être interrompue par l'un des groupes suivants : -O- ; -CO- ; et/ou porteuse d'un ou plusieurs substituants notamment : -X ; -CX₃.
- un groupe carbocyclique, saturé, insaturé ou aromatique, ayant de 3 à 8 atomes de carbone, de préférence, 6 atomes de carbone, éventuellement porteur d'un ou plusieurs atomes d'halogène, de préférence, chlore ou brome.

Parmi les groupes précisés, Rₐ et R_{b} représentent préférentiellement ;
- un groupe alkyle, linéaire ou ramifié comprenant de 1 à 12 atomes de carbone, éventuellement porteur d'un ou plusieurs atomes d'halogène,
- un groupe cyclohexyle ou phényle, éventuellement porteur d'un ou plusieurs atomes d'halogène, un groupe trihalogénométhyle,

A titre d'exemples d'anhydrides, on peut citer :
- l'anhydride acétique,
- l'anhydride propanoïque,
- l'anhydride isobutyrique,
- l'anhydride trichloroacétique,
- l'anhydride trifluoroacétique,
- l'anhydride benzoïque.
- l'anhydride de monochloroacétyle,
- l'anhydride de dichloroacétyle,
- l'anhydride pivalique.

Parmi les anhydrides précités, l'anhydride acétique est préféré.

L'invention n'exclut pas l'anhydride carboxylique soit engendré dans le milieu, à partir d'un acide carboxylique.

Comme mentionné précédemment, on fait appel dans une variante préférée du procédé de l'invention à un solvant organique.

Plusieurs impératifs président au choix du solvant organique.

Une première caractéristique du solvant organique est qu'il soit stable dans le milieu réactionnel.

Une deuxième caractéristique est que le solvant présente un point d'ébullition élevé, de préférence, supérieur ou égal à 50°C.

Comme exemples de solvants convenant à la présente invention, on peut citer en particulier les hydrocarbures aromatiques, halogénés ou non, les éther-oxydes aliphatiques, cycloaliphatiques ou aromatiques.

A titre d'exemples d'hydrocarbures aliphatiques, on peut citer plus les hydrocarbures aromatiques et plus particulièrement les hydrocarbures aromatiques comme notamment le benzène, le toluène, les xylènes, le cumène, les coupes pétrolières constituées de mélange d'alkylbenzènes notamment les coupes de type Solvesso.

En ce qui concerne les hydrocarbures halogénés aliphatiques ou aromatiques, on peut mentionner plus particulièrement, le dichlorométhane, le 1,2-dichloroéthane, le mono- ou dichlorobenzène.

On peut utiliser également à titre de solvants organiques, les éther-oxydes aliphatiques, cycloaliphatiques ou aromatiques et, plus particulièrement, l'oxyde de dipropyle, l'oxyde de diisopropyle, l'oxyde de dibutyle, le méthyltertiobutyléther, le diméthyléther de l'éthylèneglycol (ou glyme), le diméthyléther du diéthylèneglycol (ou diglyme) ; l'oxyde de phényle ; le dioxane, le tétrahydrofurane (THF).

Comme exemples de solvants organiques aprotiques, plus polaires qui peuvent également être mis en oeuvre dans le procédé de l'invention, on peut citer plus particulièrement les carboxamides linéaires ou cycliques comme le N,N-diméthylacétamide (DMAC), le N,N-diéthylacétamide, le diméthylformamide (DMF), le diéthylformamide ou la 1-méthyl-2-pyrrolidinone (NMP) ; le diméthylsulfoxyde (DMSO) ; l'hexaméthylphosphotriamide (HMPT) ; la tétraméthylurée ; les composés nitrés tels que le nitrométhane, le nitroéthane, le 1-nitropropane, le 2-nitropropane ou leurs mélanges, le nitrobenzène ; les nitriles alphatiques ou aromatiques comme l'acétonitrile, le propionitrile, le butanenitrile, l'isobutanenitrile, le benzonitrile, le cyanure de benzyle ; la tétraméthylène sulfone (sulfolane).

On peut également utiliser un mélange de solvants.

Selon le procédé de l'invention, la cyclisation du substrat de départ est conduite en présence d'une base et d'un anhydride d'acide carboxylique.

Plus précisément, la quantité de base exprimée par le rapport entre le nombre de moles de base et le nombre de moles de substrat de départ répondant préférentiellement à la formule (I) varie entre 0,05 et 1,0 et se situe de préférence, entre 0,1 et 0,2.

La quantité d'anhydride d'acide carboxylique mise en oeuvre est telle que le rapport molaire anhydride d'acide carboxylique/composé de formule (I) varie de préférence, entre 2 et 10.

Dans la variante préférée du procédé de l'invention qui consiste à mettre en oeuvre un solvant organique, la quantité d'anhydride d'acide carboxylique mise en oeuvre est telle que le rapport molaire anhydride d'acide carboxylique/composé de formule (I) varie de préférence, entre 1 et 3 et encore plus préférentiellement entre 1 et 2.

Pour ce qui est de la quantité de solvant organique à mettre en oeuvre, elle est déterminée en fonction de la nature du solvant organique choisi.

Elle est déterminée de telle sorte que la concentration du substrat dans le solvant organique soit de préférence comprise entre 1 et 10 mol/litre et plus préférentiellement entre 2 et 3 mol/litre.

La réaction de cyclisation du substrat de départ a lieu à une température qui est avantageusement située entre 50°C et 160°C, de préférence, entre 100°C et 140°C.

La réaction de cyclisation est généralement mise en oeuvre sous pression atmosphérique mais de préférence, sous atmosphère contrôlée de gaz inertes. On peut établir une atmosphère de gaz rares, de préférence l'argon mais il est plus économique de faire appel à l'azote.

D'un point de vue pratique, la réaction est simple à mettre en oeuvre.

L'ordre de mis en oeuvre des réactfs n'est pas critique. Une variante préférée consiste à charger le solvant organique si présent, le substrat, l'anhydride carboxylique et ensuite la base et l'on chauffe à la température souhaitée.

En fin de réaction, on obtient le produit cyclisé qui répond préférentiellement à la formule (V) suivante : 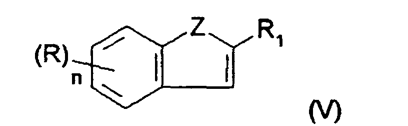 dans ladite formule (V), R, R₁, Y et n ont la signification donnée précédemment.

On récupère le composé de type benzofurane ou benzothiophène selon les techniques classiques utilisées, notamment par distillation et/ou par extraction.

On peut, par exemple, distiller le solvant organique si présent, l'anhydride d'acide carboxylique et l'acide carboxylique formé.

On peut alors introduire un solvant d'extraction du composé cyclisé. Comme exemples plus spécifiques, on peut mentionner les hydrocarbures aliphatiques ou aromatiques, halogénés ou non. On peut citer notamment le dichlorométhane, le dichloroéthane, le méthylcyclohexane ou l'éther de pétrole.

La quantité mise en oeuvre est du même ordre de grandeur que celle du solvant organique.

On sépare les phases aqueuse et organique et l'on traite cette dernière par une solution aqueuse basique, de préférence, une solution aqueuse de soude diluée (5 à 10 %) de telle sorte que le pH se situe entre 6 et 8.

On effectue de un à plusieurs lavages (par exemple 3) et l'on finit généralement par un lavage à l'eau.

On récupère le produit cyclisé après élimination du solvant organique d'extraction.

Le procédé de l'invention peut s'appliquer aussi bien à des substrats connus décrits dans la littérature qu'à des substrats nouveaux décrits ci-après et qui répondent à la formule (I') suivante : 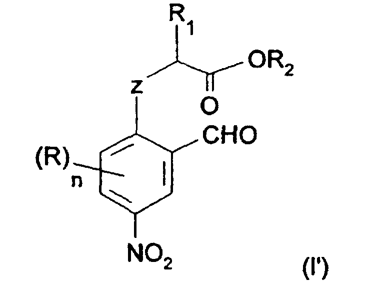 dans laquelle :
- R₁ représente un groupe alkyle linéaire ou ramifié ayant de 1 à 12 atomes de carbone, un groupe phényle éventuellement substitué par un groupe alkyle ayant de 1 à 4 atomes de carbone ou un groupe halogénophényle,
- R₂ représente un atome d'hydrogène, un groupe hydrocarboné ayant de 1 à 12 atomes de carbone qui peut être un groupe alkyle linéaire ou ramifié, un groupe cycloalkyle, un groupe phényle ou un groupe phénylalkyle,
- Z représente un atome d'oxygène ou de soufre,
- R représente un atome d'hydrogène ou un substituant,
- n est un nombre égal à 0,1, 2, ou 3, de préférence, égal à 0,
- lorsque n est supérieur à 1, deux groupes R et les 2 atomes successifs du cycle benzénique peuvent former entre eux un cycle saturé, insaturé ou aromatique ayant de 5 à 7 atomes de carbone.

Dans la formule (I'), le cycle benzénique peut porter un substituant.

Comme exemples plus particuliers de groupes R, on peut mentionner entre autres :
- un groupe hydroxyle,
- un groupe alkyle linéaire ou ramifié, ayant de 1 à 6 atomes de carbone, de préférence de 1 à 4 atomes de carbone, tel que méthyle, éthyle, propyle, isopropyle, butyle, isobutyle, sec-butyte, tert-butyle,
- un groupe alkoxy ayant de 1 à 6 atomes de carbone, de préférence de 1 à 4 atomes de carbone,
- un groupe ester ayant de 1 à 10 atomes de carbone, de préférence de 1 à 4 atomes de carbone,
- un groupe alkylamide ayant de 1 à 6 atomes de carbone, de préférence 1 à 4 atomes de carbone,
- un groupe carboxamide,
- un atome d'halogène,
- un groupe trifluorométhyle.

Les composés préférés du procédé de l'invention répondent à la formule (I') dans laquelle R représente un atome d'hydrogène, un groupe méthyle ou éthyle, un groupe méthoxy ou éthoxy.

Lorsque n est supérieur à 1, deux groupes R et les 2 atomes successifs du cycle benzénique peuvent former entre eux un cycle saturé, insaturé ou aromatique ayant de 5 à 7 atomes de carbone, de préférence 6 atomes de carbone. Ainsi, deux groupes R forment avantageusement un cycle benzénique.

Le groupe R₁ est avantageusement un groupe alkyle ayant de 1 à 4 atomes de carbone.

L'invention n'exclut pas le fait que R₂ représente un autre groupe tel que cycloalkyle, phényle ou arylalkyle mais le groupe R₂ étant éliminé, il est intéressant d'un point de vue économique qu'il soit le plus simple possible par exemple, un groupe alkyle inférieur c'est-à-dire ayant de 1 à 4 atomes de carbone R₂ peut également représenter un atome d'hydrogène ce qui correspond à la présence d'un groupe carboxylique.

Le composé nitroaromatique de formule (I') peut être obtenu selon un procédé qui consiste à effectuer la nitration sélective en position 4 d'un composé aromatique répondant à la formule (II') : 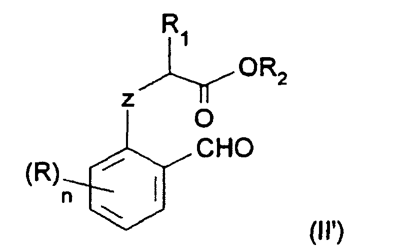 dans ladite formule (II'), R, R₁, R₂, Z et n ont la signification donnée précédemment.

Le composé aromatique de formule (II') peut être obtenu selon un procédé qui consiste à faire réagir :
- un composé de type 2-hydroxy- ou 2-thiobenzaldéhyde de formule (III') : 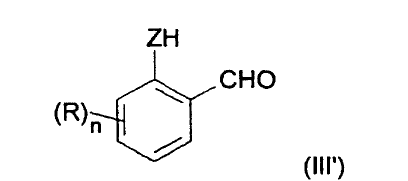 dans ladite formule (III'), R, Z et n ont la signification donnée précédemment,
- et un acide carboxylique ou dérivé de formule (IV) : 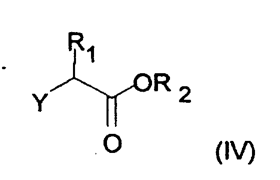 dans ladite formule (IV) :

- Y représente un groupe partant, de préférence, un atome d'halogène ou un groupe ester sulfonique de formule - OSO₂ - 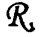 dans lequel 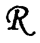 est un groupe hydrocarboné,
- R₁ , R₂ ont la signification donnée précédemment.

Dans la formule du groupe ester sulfonique, 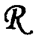 est un groupe hydrocarboné d'une nature quelconque. Toutefois, étant donné que Y est un groupe partant, il est intéressant d'un point de vue économique que 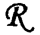 soit d'une nature simple, et représente plus particulièrement un groupe alkyle linéaire ou ramifié ayant de 1 à 4 atomes de carbone, de préférence, un groupe méthyle ou éthyie mais il peut également représenter par exemple un groupe phényle ou tolyle ou un groupe trifluorométhyle. Parmi les groupes Y, le groupe préféré est un groupe triflate ce qui correspond à un groupe 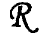 représentant un groupe trifluorométhyle.

Comme groupes partants préférés, on choisit de préférence, les atomes d'halogène, brome, chlore ou iode et de préférence un atome de brome ou de chlore.

Conformément au procédé de l'invention, on part d'un nouvel intermédiaire de fabrication répondant à la formule (I').

Il peut être obtenu selon une réaction de nitration sélective en position 4 d'un composé répondant à la formule (II') O- ou S-alkylé, par réaction de ce dernier avec une source de NO₂⁺, en présence d'acide sulfurique: la réaction pouvant être conduite ou pas dans un solvant organique.

A cet effet, on fait réagir ledit composé avec une source de NO₂⁺.

Ainsi, on peut partir du dioxyde d'azote NO₂, de l'anhydride azoteux N₂O₃, du peroxyde d'azote N₂O₄, de l'oxyde d'azote NO associé à un agent oxydant tel que, par exemple, l'acide nitrique, le dioxyde d'azote ou l'oxygène. Dans le cas où le réactif est gazeux dans les conditions réactionnelles, on le fait buller dans le milieu.

On peut également faire appel à l'acide nitreux, à un sulfate de nitrosyle ou nitrose ou à un sel nitreux, de préférence un sel de métal alcalin, et encore plus préférentiellement, le sodium associé à un agent oxydant, de préférence l'acide nitrique.

Il est également possible de mettre en oeuvre des nitrites d'alkyle associés à un agent oxydant et plus particulièrement ceux répondant à la formule (VII) :

Rₐ- ONO (VII)

dans ladite formule (VII), Rₐ représente un groupe alkyle linaire ou ramifié ayant de 1 à 12 atomes de carbone, de préférence, de 1 à 4 atomes de carbone.

La quantité de source de NO₂⁺ est au moins égale à la quantité stoechiométrique du composé aromatique O- ou S-alkylé. Ainsi, le rapport entre le nombre de moles de source de NO₂⁺ et le nombre de moles de composé aromatique O- ou S-alkylé est compris avantageusement entre 1,0 et 1,2.

On fait appel préférentiellement à une solution d'acide nitrique concentrée ayant une concentration préférentielle comprise entre 70 et 99 %.

Comme mentionné précédemment, la source de NO₂⁺ est associée à l'acide sulfurique.

Une variante du procédé de l'invention consiste à faire appel à un mélange sulfonitrique (mélange d'acide nitrique et d'acide sulfurique comprenant de 50 à 98 % en poids d'acide nitrique).

La quantité d'acide nitrique exprimée par le rapport molaire composé aromatique O- ou S-alkylé/acide nitrique varie généralement entre 0,9 et 1,1, de préférence, entre 0,95 et 1,05.

La quantité d'acide sulfurique exprimée par le rapport molaire composé aromatique O- ou S-alkylé/acide sulfurique varie généralement entre 0,9 et 1,1, de préférence, entre 0,95 et 1,05.

La concentration en acide sulfurique est avantageusement comprise entre 50 % et 98 %.

A cet effet, on fait appel à l'acide nitrique ou à un précurseur de l'acide nitrique tel que par exemple le peroxyde d'azote.

La réaction de nitration peut être éventuellement conduite dans un solvant organique qui soit inerte dans les conditions réactionnelles.

Comme exemples plus particuliers de solvants organiques, on peut citer les hydrocarbures halogénés aliphatiques et plus particulièrement, les hydrocarbures perchlorés tels que notamment le tétrachlorométhane, l'hexachloroéthane ; les hydrocarbures partiellement chlorés tels que le dichlorométhane le 1,2-dichloroéthane.

Le dichlorométhane est le solvant préféré.

En ce qui concerne la concentration du composé aromatique O- et S-alkylé, dans le milieu réactionnel, elle est de préférence comprise entre 0,2 et 3 mol/l et de préférence entre 0,3 et 1,5 mol/l.

Celui-ci est introduit généralement sous forme liquide.

La réaction est avantageusement effectuée à une température se situant entre - 5°C et 10°C et sous atmosphère de gaz inertes.

Le procédé de l'invention est généralement mis en oeuvre sous pression atmosphérique.

Selon une variante préférée du procédé de l'invention, on conduit l'étape de nitration sous atmosphère contrôlée de gaz inertes. On peut établir une atmosphère de gaz rares, de préférence l'argon mais il est plus économique de faire appel à l'azote.

Plusieurs modes de mises en oeuvre peuvent être envisagés.

Une première variante consiste à charger d'abord la solution d'acide sulfurique puis d'ajouter ensuite en parallèle le composé aromatique O- ou S-alkylé et l'acide nitrique.

Selon une autre variante, on introduit la solution d'acide sulfurique et l'acide nitrique puis l'on ajoute le composé aromatique O- ou S-alkylé, de préférence par portions ou de manière continue par coulée.

Une autre variante réside dans le fait d'introduire en parallèle, sur un pied de cuve, le composé aromatique O- ou S-alkylé d'un côté et l'acide sulfurique et l'acide nitrique de l'autre.

La réaction dure avantageusement entre 3 et 10 heures.

En fin de réaction, on obtient le produit désiré répondant à la formule (I').

On récupère le produit selon les techniques classiques utilisées dans le domaine technique.

On peut en particulier effectuer une hydrolyse à l'eau, de préférence, avec de la glace mise en oeuvre à raison par exemple de 100 à 150 % du poids du composé de formule (I').

On obtient un solide qui est séparé selon les techniques classiques de séparation solide/liquide, de préférence, par filtration.

On obtient ainsi le produit souhaité.

Le composé de formule (II') qui intervient dans la préparation du composé de formule (I') peut être obtenu notamment selon une réaction de O- ou S-alkylation d'un composé de type 2-hydroxy- ou 2-thiobenzaldéhyde de formule (III') avec un acide carboxylique ou dérivé de formule (IV).

Un mode de réalisation consiste à faire réagir un composé aromatique de formule (III') avec un acide carboxylique ou dérivé de formule (IV) : la réaction étant conduite en présence d'une base, de préférence dans un solvant organique.

Parmi les composés de formule (III'), l'aldéhyde salicylique est préféré.

Pour ce qui est du composé de formule (IV), on fait appel préférentiellement au 2-bromohexanoate de méthyle ou d'éthyle.

Le rapport molaire entre le composé de formule (III') et le composé de formule (IV) est avantageusement choisi entre 1 et 1,2.

Conformément au procédé de l'invention, on fait réagir le composé de type 2-hydroxy- ou 2-thiobenzaldéhyde de formule (III') sous forme salifiée et l'acide carboxylique ou dérivé de formule (IV), dans un solvant organique tel que défini.

On peut faire appel à une forme salifiée d'un composé de type 2-hydroxy-ou 2-thiobenzaldéhyde préparée extemporanément mais il est également possible de le préparer in situ en faisant réagir le composé de type 2-hydroxy-ou 2-thiobenzaldéhyde et la base.

Intervient donc, dans le procédé de l'invention, une base qui peut être minérale ou organique.

Conviennent particulièrement bien à la mise en oeuvre du procédé de l'invention, les bases minérales telles que les sels de métaux alcalins ou alcalino-terreux, de préférence, un hydroxyde de métal alcalin ou alcalino-terreux qui peut être l'hydroxyde de sodium, de potassium ou de calcium ; un carbonate ou hydrogénocarbonate d'un métal alcalin, de préférence, le carbonate de sodium.

Il est également possible de faire appel à une base organique telle qu'un hydroxyde d'ammonium quaternaire ou à une amine.

Comme exemples d'hydroxyde d'ammonium quaternaire, on met en oeuvre préférentiellement, les hydroxydes de tétralkylammonium ou de trialkylbenzylammonium dont les groupes alkyle identiques ou différents représentent une chaîne alkyle linéaire ou ramifiée ayant de 1 à 12 atomes de carbone, de préférence de 1 à 6 atomes de carbone.

On choisit préférentiellement l'hydroxyde de tétraméthylammonium, l'hydroxyde de tétraéthylammonium ou l'hydroxyde de tétrabutylammonium.

Il est également possible selon l'invention d'avoir recours à des hydroxydes de trialkylbenzylammonium et notamment à l'hydroxyde de triméthylbenzylammonium.

Comme exemples d'amines, on peut mentionner entre autres les amines tertiaires.

Parmi les bases utilisables, il convient de citer les amines tertiaires et plus particulièrement celles répondant à la formule générale (VIII)

N - (R₃)₃ (VIII)

dans laquelle :
- les groupes R₃, identiques ou différents, représentent des restes hydrocarbonés ayant de 1 à 20 atomes de carbone, tels que des groupes alkyle, cycloalkyle, aryle ou hétérocyclique ;
- 2 groupes R₃ forment ensemble et avec l'atome d'azote un hétérocycle ayant 4 à 6 atomes.

Plus particulièrement :
- les symboles R₃ représentent un groupe alkyle ayant 1 à 10 atomes de carbone et de préférence 1 à 4 atomes de carbone ou un groupe cyclopentyle ou cyclohexyle ou un groupe pyridinyle ;
- 2 groupes R₃ forment ensemble et avec l'atome d'azote un cycle pipéridine ou pyrrolidine.

A titre d'exemples de telles amines, on peut citer la triéthylamine, la tri-*n*-propylamine, la tri-*n*-butylamine, la méthyldibutylamine, la méthyldicyclohexylamine, l'éthyldiisopropylamine, la N,N-diéthylcyclohexylamine, la diméthylamino-4 pyridine, la N-méthylpipéridine, la N-éthylpipéridine, la N-*n*-butylpipéridine, la 1,2-diméthylpipéridine, la N-méthylpyrrolidine, la 1,2-diméthylpyrrolidine.

Pour des considérations économiques, on choisit parmi toutes les bases, préférentiellement le carbonate de sodium ou de potassium.

Bien que la forme de mise en oeuvre de la base est sous forme solide, on peut également faire appel à une base en solution. La concentration de la solution basique de départ n'est pas critique. La solution d'hydroxyde de métal alcalin mise en oeuvre a une concentration généralement comprise entre 10 et 50 % en poids.

La quantité de base introduite dans le milieu réactionnel tient compte de la quantité nécessaire pour salifier la fonction hydroxyle ou thiol du composé de type 2-hydroxy- ou 2-thiobenzaldéhyde.

Généralement, la quantité de base exprimée par rapport au composé de type 2-hydroxy- ou 2-thiobenzaldéhyde varie entre 90 et 120 % de la quantité stoechiométrique.

On peut effectuer la salification du groupe hydroxyle ou thiol du substrat de départ de formule (III') éventuellement dans une étape préalable. On peut donc salifier le composé de formule (III') soit en introduisant la base puis en la faisant réagir à une température avantageusement comprise entre 0°C et 100°C, de préférence, entre 25°C et 50°C, soit en introduisant la base en même temps que le composé de formule (IV)

Conformément à l'invention, la réaction de O- ou S-alkylation est conduite avantageusement en phase liquide comprenant le composé de formule (III') et le composé de formule (IV), en présence d'une base.

L'un des réactifs de départ peut servir de solvant réactionnel mais il est également possible de faire appel à un solvant organique.

On choisit un solvant organique, moins activé que le substrat de départ et qui de préférence le solubilise.

Comme exemples de solvants convenant à la présente invention, on peut citer en particulier les hydrocarbures aromatiques, halogénés ou non, les éther-oxydes aliphatiques, cycloaliphatiques ou aromatiques. Des exemples de tels solvants sont donnés ci-dessus.

Comme exemples de solvants organiques aprotiques, plus polaires qui peuvent également être mis en oeuvre dans le procédé de l'invention, on peut citer plus particulièrement les nitriles alphatiques ou aromatiques comme l'acétanitrile, le propionitrile, le benzonitrile ; les carboxamides linéaires ou cycliques comme le N,N-diméthylacétamide (DMAC), le diméthylformamide (DMF), le diéthylformamide ou la 1-méthyl-2-pyrrolidinone (NMP).

Les solvants préférés sont le DMAC ou le DMF.

On peut également utiliser un mélange de solvants organiques.

En ce qui concerne la concentration du composé de type 2-hydroxy- ou 2-thiobenzaldéhyde dans le milieu réactionnel, elle est de préférence comprise entre 2 % et 50 % en poids.

Une variante du procédé de l'invention consiste à ajouter des ions iodure afin d'accélérer la réaction. Ainsi, on peut faire appel notamment à des iodures de métaux alcalins, de préférence l'iodure de potassium ou à des iodures de tétralkylammonium, de préférence l'iodure de tétrabutylammonium.

La quantité de iodure mise en jeu, exprimée par le rapport entre le nombre de moles de sel iodé et le nombre de moles de composé de formule (III') peut varier entre 0,05 et 0,2.

La température de la réaction du composé aromatique de formule (III') avec un acide carboxylique ou dérivé de formule (IV) est avantageusement comprise entre 0°C et 100°C, de préférence, entre 25°C et 50°C.

La réaction a lieu généralement sous pression atmosphérique.

Selon une variante préférée du procédé de l'invention, on conduit le procédé de l'invention, sous atmosphère contrôlée de gaz inertes. On peut établir une atmosphère de gaz rares, de préférence l'argon mais il est plus économique de faire appel à l'azote.

D'un point de vue pratique, le procédé est simple à mettre en oeuvre.

Un mode de réalisation de l'invention consiste à charger tous les réactifs, la base, le solvant organique éventuellement les ions iodure.

On porte le milieu à la température réactionnelle choisie.

Comme mentionné précédemment, on peut effectuer la salification dans une étape préalable et introduire le composé de formule (III') la base et le solvant organique, porter le milieu à la température choisie puis ajouter le composé de formule (IV) et éventuellement des ions iodure puis on chauffe.

On obtient le produit désiré qui répond à la formule (II').

On récupère le produit obtenu d'une manière classique.

On peut par exemple éliminer les sels formés au cours de la réaction par addition d'eau et extraire ledit produit en phase organique, dans un solvant adéquat par exemple l'éther isopropylique.

Le solvant organique peut être éliminé d'une manière classique par évaporation.

On effectue la cyclisation du composé de formule (I') selon le procédé de l'invention pour obtenir un composé hétérocyclique répondant à la formule générale (V') : 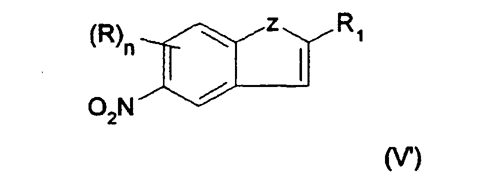 dans la formule (V'), R, R₁, Z et n ont la signification donnée précédemment.

Quand le composé de formule (I') comprend à titre de groupe COOR₂, une fonction ester au lieu d'une fonction carboxylique, il est nécessaire d'effectuer au préalable une étape de saponification, avant d'effectuer la cyclisation. Selon une autre variante, il est possible d'effectuer de la même manière, la saponification si nécessaire du composé de formule (II'), avant l'opération de nitration.

A cet effet, on fait donc réagir le composé de formule (I') avec une base en milieu hydro-organique.

Comme base, on fait appel préférentiellement à la soude ou à la potasse qui sont mises en oeuvre sous la forme de paillettes ou de solutions concentrées, par exemple à 40 % pour la soude.

La quantité de base mise en oeuvre exprimée par le rapport entre le nombre de moles du composé de formule (I') et le nombre de moles de base est choisie de préférence entre 1 et 5 et plus préférentiellement entre 1 et 2.

La base est solubilisée en milieu aqueux ou hydro-organique.

On fait appel de préférence à un solvant organique polaire.

Comme exemples de solvants organiques convenant à la présente invention, on peut citer plus particulièrement les alcools aliphatiques tels que l'éthanol, le propanol, le butanol, le pentanol, l'éthylène glycol ; les alcools cycloaliphatiques, notamment le cyclohexanol et les alcools arylaliphatiques, plus particulièrement l'alcool benzylique. On peut également envisager les éthers monométhylique, monoéthylique, monopropylique, monobutylique, de l'éthylène glycol vendus sous la dénomination commerciale de Cellosolves®.

La concentration du composé de formule (I') dans le milieu réactionnel (eau + solvants organiques) varie avantageusement entre 5 et 50 %, de préférence, entre 5 et 20 % en poids.

Le rapport volumique entre le solvant organique et l'eau peut varier, par exemple, entre 0,1 et 0,9, et il se situe de préférence, vers 0,1 et 0,2.

Le choix du solvant organique et du rapport eau/solvant organique est déterminé de telle sorte que la solution obtenue soit homogène.

On effectue la réaction de saponification à une température comprise entre la température ambiante et la température de reflux du mélange réactionnel, préférentiellement à une température voisine de 50°C. Par température ambiante, on entend généralement une température comprise entre 15°C et 25°C.

Selon un mode de réalisation pratique de l'invention, on introduit le composé de formule (I') dans le milieu aqueux ou hydro-organique puis l'on additionne la base puis l'on amène le milieu réactionnel à la température choisie.

En fin de réaction, si nécessaire, on neutralise l'excès de base par une solution acide, de préférence, une solution d'acide minéral ou d'un sel minéral, tel que par exemple, l'acide chlorhydrique ou le chlorure d'ammonium.

Le produit obtenu précipite puis on le sépare selon les techniques classiques de séparation solide/liquide, de préférence, par filtration.

On effectue alors la cyclisation conformément au procédé de l'invention.

On obtient un dérivé de type benzofurane ou benzothiophène nitré en position 5 et répondant à la formule (V').

Le procédé de l'invention est particulièrement adapté à la préparation du 2-n-butyl-5-nitrobenzofurane.

On donne ci-après des exemples de réalisation de l'invention. Ces exemples sont donnés à titre illustratif et sans caractère limitatif.

### Exemple 1

### Préparation du 5-nitro-2-butylbenzofuranne.

Dans un réacteur de 200 ml contenant 80 g d'anhydride acétique on charge 23,4 g d'acide 2-(2-formyl-4-nitro-phénoxy)hexanoïque et 2,3 g de carbonate de potassium.

Le mélange réactionnel est chauffé sous atmosphère d'azote au reflux, à une température voisine de 130°C, pendant 5 heures.

Après retour à une température voisine de 25°C, le milieu est extrait par 15 fois 50 ml d'éther de pétrole (40-65 °C).

Les phases organiques collectées sont lavées par 300 ml d'une solution aqueuse d'hydrogénocarbonate de sodium (10 %) et séchées sur sulfate de magnésium.

On obtient ainsi, après évaporation des solvants sous pression réduite, 11,2 g de 5-nitro-2-butylbenzofuranne (RR pondéral = 62 %) sous forme d'une huile jaune (pureté RMN = 95 %)

Son spectre RMN est le suivant :
RMN ¹H (DMSO-d6) : δ 0,91 (t, 3H, CH₃) ; 1,37 (m, 2H, CH₂-CH₃) ; 1,67 (m, 2H, CH₂-CH₂-CH₃) ; 2,81 (m, 2H, CH₂-C=) ; 6,81 (s, 1H, CH=) ; 7,71 (d, J= 9 Hz, 1H, ArH) ; 8,12 (dd, J= 9 Hz, J= 2,5 Hz, 1H, ArH) ; 8,47 (d, J= 2,5 Hz, 1H, ArH). La pureté du produit est estimée supérieure à 95 % molaire.

### Exemple 2

### Préparation du 5-nitro-2-butylbenzofuranne, en présence d'un solvant organique.

Dans un réacteur de 200 ml contenant 40,9 g de diméthylformamide et 25,1 g d'acide 2-(2-formyl-4-nitro-phénoxy)-hexanoïque (0,1 mol), on charge 15,3 g d'anhydride acétique (0,15 mol), et 2,63 g de carbonate de potassium (0,019 mol).

Le milieu réactionnel est chauffé sous atmosphère d'azote à une température voisine de 135°C pendant 5 heures.

Après retour à une température voisine de 25°C, le milieu est chauffé progressivement entre 40°C et 120°C sous 23 mbar pour distiller le diméthylformamide, l'acide acétique et l'anhydride acétique.

Il reste dans le réacteur une masse réactionnelle épaisse mais agitable. A ce milieu, est ajouté 50 ml de dichlorométhane.

L'ensemble est lavé successivement par 3 fois 25 ml d'une solution aqueuse de soude à 5 % puis 25 ml d'eau.

Le dichlorométhane est distillé à pression atmosphérique pour conduire à 15,8 g de 5-nitro-2-butylbenzofuranne (RR pondéral = 72,1 %) sous la forme d'une huile jaune.

La pureté déterminée par RMN est de 95 % et par chromatographie en phase gazeuse de 99,6 %.

Son spectre RMN est le suivant :
RMN ¹H (DMSO-d6) : δ 0,91 (t, 3H, CH₃) ; 1,37 (m, 2H, CH₂-CH₃) ; 1,67 (m, 2H, CH₂-CH₂-CH₃) ; 2,81 (m, 2H, CH₂-C=) ; 6,81 (s, 1H, CH=) ; 7,71 (d, J= 9 Hz, 1H, ArH) ; 8,12 (dd, J= 9 Hz, J= 2,5 Hz, 1H, ArH) ; 8,47 (d, J= 2,5 Hz, 1H, ArH).

### Exemple 3

### Préparation de l'acide 2-(2-formyl-4-nitro-phénoxy)-hexanoïque.

L'acide 2-(2-formyl-4-nitro-phénoxy)-hexanaïque peut être préparé de la manière suivante :

Dans un réacteur tétracol de 250 ml, muni d'une agitation pale Téflon demi lune, d'un thermomètre, d'une ampoule d'addition de 50 ml, d'un réfrigérant à serpentin et d'une arrivée d'azote, on charge successivement 29,5 g de 2-(2-formyl-4-nitro-phénoxy)-hexanoate de méthyle, 148 ml d'eau.

On ajoute en 20 minutes, 8,4 g d'une solution aqueuse à 50 % d'hydroxyde de sodium.

Après 15 minutes sous agitation à une température voisine de 25°C, le milieu réactionnel est chauffé pendant 2 heures aux environs de 50°C.

Le milieu limpide obtenu de couleur rouge est partiellement évaporé (50 ml) sous pression réduite (20 mm Hg) pour éliminer le méthanol formé puis redilué par 50 ml d'eau.

Le pH du milieu réactionnel est amené au voisinage de 1,8 par addition lente de 10,8 g d'acide chlorhydrique concentré en maintenant la température au voisinage de 45°C sous agitation.

Après une heure sous agitation, la température du milieu est portée au voisinage de 55°C pendant 20 minutes puis laissé à température ambiante pendant 12 heures.

Le produit solide est séparé par filtration sur verre fritté N°3 et lavé par 2 fois 50 ml d'eau puis séché à l'étuve pendant 12 heures à une température voisine de 55°C.

On obtient ainsi 26,8 g d'acide 2-(2-formyl-4-nitro-phénoxy)-hexanoïque sous forme d'un solide jaune clair fondant à 110-111°C et titrant 97,5 % par dosage potentiométrique.

Son spectre RMN est le suivant :
RMN ¹H (DMSO-d6) : δ 0,91 (t, 3H, CH₃) ; 1,38 (m, 2H, CH₂-CH₃) ; 1.51 (m, 2H, CH₂-CH₂-CH₃) ; 2,02 (m, 2H, CH₂-CH) ; 5,24 (t, 1H, CH) ; 7,34 (d, J= 9 Hz, 1H, ArH) ; 8,44 (d, J= 2 Hz, 1H, ArH) ; 8,47 (dd, J= 9 Hz, J= 2 Hz, 1H, ArH) ; 10,42 (s, 1H, CHO) ; 13,45 (pic large, 1H, COOH).

### Exemple 4

### Préparation de l'acide 2-(2-formyl-phénoxy)-hexanoïque.

L'acide 2-(2-formyl-phénoxy)-hexanoïque peut être préparé de la manière suivante :

Dans un réacteur tétracol de 250 ml, muni d'une agitation pale Téflon demi lune, d'un thermomètre, d'une ampoule d'addition de 50 ml, d'un réfrigérant à serpentin et d'une arrivée d'azote, on charge successivement 29,5 g de 2-(2-formyl-phénoxy)-hexanoate de méthyle, 148 ml d'eau.

On ajoute en 20 minutes, 10.4 g d'une solution aqueuse à 50 % d'hydroxyde de sodium.

Après 15 minutes sous agitation à une température voisine de 25°C, le milieu réactionnel est chauffé pendant 2 heures aux environs de 50°C.

Le milieu limpide obtenu est partiellement évaporé (50 ml) sous pression réduite (20 mm Hg) pour éliminer le méthanol formé puis redilué par 50 ml d'eau.

Le pH du milieu réactionnel est amené au voisinage de 1,8 par addition lente de 10,8 g d'acide chlorhydrique concentré en maintenant la température au voisinage de 45°C sous agitation.

Après une heure sous agitation, la température du milieu est portée au voisinage de 55°C pendant 20 minutes puis laissé à température ambiante pendant 12 heures.

Le produit solide est séparé par filtration sur verre fritté N°3 et lavé par 2 fois 50 ml d'eau puis séché à l'étuve pendant 12 heures à une température voisine de 55°C.

On obtient ainsi 27.4 g d'acide 2-(2-formyl-phénoxy)-hexanoïque sous forme d'un solide jaune clair et titrant 98 % par dosage potentiométrique.

### Exemple 5

### Préparation du 2-(2-formyl-4-nitro-phénoxy)-hexanoate de méthyle.

Le 2-(2-formyl-4-nitro-phénoxy)-hexanoate de méthyle peut être préparé de la manière suivante :

Dans un ballon tétracol de 250 ml, muni d'une agitation pale Téflon demi lune, d'un thermomètre, d'une ampoule d'addition de 50 ml, d'un réfrigérant à serpentin et d'une arrivée d'azote, on charge 123 g d'acide sulfurique concentré à 96 %.

Le milieu réactionnel est refroidi à une température voisine de 5 °C puis on ajoute à cette même température 30 g (0,12 mole) de 2-(2-formyl-phénoxy)-hexanoate de méthyle.

Après 15 minutes sous agitation, on additionne en 2 heures, 15,9 g (0,126 mol) de sulfonitrique (50/50) en maintenant la température du milieu réactionnel au voisinage de 5°C puis on ajoute en 30 minutes 76,9 g de glace conduisant à un titre de 60% en H₂SO₄.

Le mélange réactionnel est filtré sur verre fritté N° 3 après 10 minutes d'agitation.

Le produit brut obtenu est dissous dans 100 ml de dichlorométhane, et lavé avec 2 fois 50 ml d'eau.

La phase organique décantée est concentrée à l'évaporateur rotatif de 20 à 70° C sous 20 mm de mercure (durée : 2 heures).

On obtient ainsi 32,7 g de produit solide jaune beige soit un rendement en 2-(2-formyl-4-nitro-phénoxy)-hexanoate de méthyle de 92,4 % titrant 96,7 % par chromatographie en phase gazeuse.

Son spectre RMN est le suivant :
RMN ¹H (DMSO-d6) : δ 0,91 (t, 3H, CH₃) ; 1,38 (m, 2H, CH₂-CH₃) ; 1,51 (m, 2H, CH₂-CH₂-CH₃) ; 2,02 (m, 2H, CH₂-CH) ; 5,24 (t, 1H, CH) ; 7,34 (d, J= 9 Hz, 1H, ArH) ; 8,44 (d, J= 2 Hz, 1H, ArH) ; 8,47 (dd, J= 9 Hz, J= 2 Hz, 1H, ArH) ; 10,42 (s, 1H, CHO) ; 13,45 (pic large, 1H, COOH).

### Exemple 6

### Préparation de l'acide 2-(2-formyl-4-nitro-phénoxy)-hexanoïque.

L'acide 2-(2-formyl-4-nitro-phénoxy)-hexanoïque peut être préparé de la manière suivante :

Dans un ballon tétracol de 250 ml, muni d'une agitation pale Téflon demi lune, d'un thermomètre, d'une ampoule d'addition de 50 ml, d'un réfrigérant à serpentin et d'une arrivée d'azote, on charge 123 g d'acide sulfurique concentré à 96 %.

Le milieu réactionnel est refroidi à une température voisine de 5 °C puis on ajoute à cette même température 28.4 g (0,12 mole) d'acide 2-(2-formyl-phénoxy)-hexanoïque.

Après 15 minutes sous agitation, on additionne en 2 heures, 15,9 g (0,126 mol) de sulfonitrique (50/50) en maintenant la température du milieu réactionnel au voisinage de 5°C puis on ajoute en 30 minutes 76,9 g de glace conduisant à un titre de 60% en H₂SO₄.

Le mélange réactionnel est filtré sur verre fritté N° 3.

Le solide obtenu est dissous dans 100 ml de dichlorométhane, et lavé par 2 fois 50 ml d'eau.

La phase organique décantée est concentrée à l'évaporateur rotatif de 20 à 70° C sous 20 mm de mercure (durée : 2 heures).

On obtient ainsi 32,1 g de produit solide jaune beige soit un rendement en acide 2-(2-formyl-4-nitro-phénoxy)-hexanoïque de 95 % titrant 97,0 % par chromatographie en phase liquide.

Son spectre RMN est le suivant :
RMN ¹H (DMSO-d6) : δ 0,91 (t, 3H, CH₃) ; 1,38 (m, 2H, CH₂-CH₃) ; 1,51 (m, 2H, CH₂-CH₂-CH₃) ; 2,02 (m, 2H, CH₂-CH) ; 5,24 (t, 1H, CH) ; 7,34 (d, J= 9 Hz, 1H, ArH) ; 8,44 (d, J= 2 Hz, 1H, ArH) ; 8,47 (dd, J= 9 Hz, J= 2 Hz, 1H, ArH) ; 10,42 (s, 1H, CHO) ; 13,45 (pic large, 1H, COOH).

### Exemple 7

### Préparation du 2-(2-formyl-phénoxy)-hexanoate de méthyle.

Le 2-(2-formyl-phénoxy)-hexanoate de méthyle peut-être préparé de la manière suivante :

Dans un réacteur de 1 litre tétracol muni d'une agitation pale demi lune, d'un thermomètre, d'un réfrigérant à serpentin et d'une ampoule d'addition de 500 ml , on charge successivement sous atmosphère contrôlée 87,1 g (0,714 mol) d'aldéhyde salicylique, 158,2 g (0,756 mol) de 2-bromo-hexanoate de méthyle, 103,5 g (0,75 mol) de carbonate de potassium et 5,9 g (0,0355 mol) d'iodure de potassium.

On ajoute 400 g de diméthylformamide et l'ensemble est chauffé sous agitation à une température voisine de 80° C pendant 4 h.

Après retour à une température voisine de 25°C, le mélange réactionnel est filtré sur verre fritté n° 3 et lavé par 50 g de diméthylformamide.

Le filtrat est concentré par évaporation sous pression réduite (25-40 mbars) puis dilué par 100 ml d'eau et extrait, successivement, par 1 fois 100 ml de dichlorométhane et 50 ml de dichlorométhane.

Les phases organiques réunies sont lavées avec 50 ml d'eau et concentrées
à sec par évaporation sous pression réduite.

On obtient ainsi 176,1 g de liquide jaune limpide correspondant à un rendement de 98,6 % en 2-[2-(formyl-phénoxy)]-hexanoate de méthyle titrant 99,6 % de pureté par chromatographie en phase gazeuse.

### Exemple 8

### Préparation du 2-(2-formyl-phénoxy)-hexanoate de méthyle.

Le 2-(2-formyl-phénoxy)-hexanoate de méthyle peut-être préparé de la manière suivante :

Dans un réacteur de 2 litres tétracol muni d'une agitation pale demi lune, d'un thermomètre, d'un réfrigérant à serpentin et d'une ampoule d'addition de 1000 ml , on charge successivement sous atmosphère contrôlée 130,6 g (1,071 mol) d'aldéhyde salicylique, 237,3 g (1,134 mol) de 2-bromo-hexanoate de méthyle, 155,2 g (1,125 mol) de carbonate de potassium.

On ajoute 600 g de diméthylformamide et l'ensemble est chauffé sous agitation à une température voisine de 80° C pendant 4 h.

Après retour à une température voisine de 25°C, le mélange réactionnel est filtré sur verre fritté n° 3 et lavé par 75 g de diméthylformamide.

Le filtrat est concentré par évaporation sous pression réduite (25-40 mbars) puis dilué par 150 ml d'eau et extrait, successivement, par 1 fois 150 ml de dichlorométhane et 75 ml de dichlorométhane.

Les phases organiques réunies sont lavées avec 75 ml d'eau et concentrées à sec par évaporation sous pression réduite.

On obtient ainsi 265 g de liquide jaune limpide correspondant à un rendement de 98.9 % en 2-[2-(formyl-phénoxy)]-hexanoate de méthyle titrant 99,6 % de pureté par chromatographie en phase gazeuse.

## Revendications

1. Procédé de préparation d'un composé de type benzofurane ou benzothiophène à partir d'un composé aromatique comprenant un cycle benzénique porteur d'une part, d'une chaîne latérale comprenant au moins deux atomes de carbone, l'un des atomes de carbone étant relié au cycle benzénique par un atome d'oxygène ou de soufre, l'autre atome de carbone est sous forme carboxylique et d'autre part d'un groupe formyle en position ortho par rapport à ladite chaîne. **caractérisé par le fait qu'**il consiste à effectuer la cyclisation de ce dernier, dans un milieu comprenant un anhydride d'acide carboxylique et en présence d'une base choisie parmi les carbonates et/ou hydrogénocarbonates métalliques ou d'ammonium.

2. Procédé selon la revendication 1 **caractérisé par le fait que** le composé aromatique de départ répond à la formule suivante : 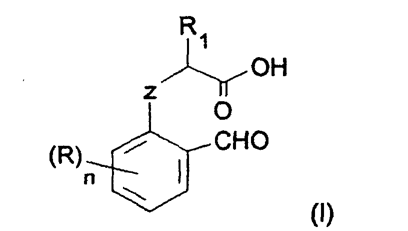 dans ladite formule (I) :
- R₁ représente un atome d'hydrogène, un groupe alkyle linéaire ou ramifié ayant de 1 à 12 atomes de carbone, un groupe phényle éventuellement substitué par un groupe alkyle ayant de 1 à 4 atomes de carbone ou un groupe halogénophényle,
- Z représente un atome d'oxygène ou de soufre,
- R représente un atome d'hydrogène ou un substituant,
- n est un nombre égal à 0,1, 2, ou 3, de préférence, égal à 0,
- lorsque n est supérieur à 1, deux groupes R et les 2 atomes successifs du cycle benzénique peuvent former entre eux un cycle saturé, insaturé ou aromatique ayant de 5 à 7 atomes de carbone.

3. Procédé selon la revendication 2 **caractérisé par le fait que** R représente un atome d'hydrogène ou l'un des groupes suivants :
- un groupe nitro,
- un groupe hydroxyle,
- un groupe alkyle linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
- un groupe alkoxy ayant de 1 à 6 atomes de carbone, de préférence de 1 à 4 atomes de carbone,
- un groupe ester ayant de 1 à 10 atomes de carbone, de préférence de 1 à 4 atomes de carbone,
- un groupe alkylamide ayant de 1 à 6 atomes de carbone, de préférence 1 à 4 atomes de carbone,
- un groupe carboxamide,
- un atome d'halogène,
- un groupe trifluorométhyle.

4. Procédé selon la revendication 1 **caractérisé par le fait que** le composé aromatique de départ répond à la formule suivante : 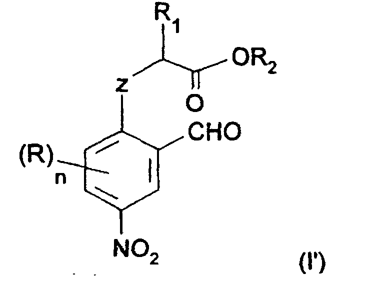 dans laquelle :
- R₁ représente un groupe alkyle linéaire ou ramifié ayant de 1 à 12 atomes de carbone, un groupe phényle éventuellement substitué par un groupe alkyle ayant de 1 à 4 atomes de carbone ou un groupe halogénophényle,
- R₂ représente un atome d'hydrogène, un groupe hydrocarboné ayant de 1 à 12 atomes de carbone qui peut être un groupe alkyle linéaire ou ramifié, un groupe cycloalkyle, un groupe phényle ou un groupe phénylalkyle,
- Z représente un atome d'oxygène ou de soufre,
- R représente un atome d'hydrogène ou un substituant,
- n est un nombre égal à 0,1, 2, ou 3, de préférence, égal à 0,
- lorsque n est supérieur à 1, deux groupes R et les 2 atomes successifs du cycle benzénique peuvent former entre eux un cycle saturé, insaturé ou aromatique ayant de 5 à 7 atomes de carbone.

5. Procédé selon l'une des revendications 2 et 4 **caractérisé par le fait que** R représente un atome d'hydrogène, un groupe méthyle ou éthyle, un groupe méthoxy ou éthoxy.

6. Procédé selon l'une des revendications 2 et 4 **caractérisé par le fait que** R₁ représente un groupe alkyle ayant de 1 à 4 atomes de carbone.

7. Procédé selon la revendication 1 **caractérisé par le fait que** la base utilisée est le carbonate de sodium ou le carbonate de potassium.

8. Procédé selon la revendication 1 **caractérisé par le fait que** l'anhydride d'acide carboxylique répond à la formule suivante : 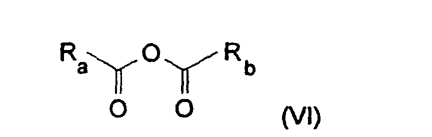 dans ladite formule (VI) :
- Rₐ et R_{b}, identiques ou différents, représentent un groupe hydrocarboné monovalent, substitué ou non qui peut être un groupe aliphatique acyclique, saturé ou insaturé, linéaire ou ramifié ; un groupe carbocyclique saturé, insaturé ou aromatique, monocyclique,
- Rₐ et R_{b} pouvant former ensemble un groupe divalent aliphatique saturé ou insaturé, linéaire ou ramifié, ayant au moins 2 atomes de carbone.

9. Procédé selon la revendication 8 **caractérisé par le fait que** l'anhydride carboxylique est un anhydride cyclique ayant de 5 à 10 atomes dans le cycle ne comprenant pas ou comprenant une double liaison, l'un des atomes pouvant être remplacé par un atome d'oxygène.

10. Procédé selon la revendication 8 **caractérisé par le fait que** l'anhydride carboxylique est un anhydride non cyclique répondant à la formule (VI), dans laquelle les groupes Rₐ et R_{b}, identiques ou différents représentent :
- un groupe aliphatique acyclique linéaire ou ramifié ayant de préférence de 1 à 24, de préférence, de 1 à 12 atomes de carbone, saturé ou comprenant une à plusieurs insaturations sur la chaîne, généralement, 1 à 3 insaturations qui peuvent être des doubles liaisons simples : la chaîne hydrocarbonée pouvant être interrompue par l'un des groupes suivants : -O- ; -CO- ; et/ou porteuse d'un ou plusieurs substituants notamment : -X ; -CX₃.
- un groupe carbocyclique, saturé, insaturé ou aromatique, ayant de 3 à 8 atomes de carbone, de préférence, 6 atomes de carbone, éventuellement porteur d'un ou plusieurs atomes d'halogène, de préférence, chlore ou brome.

11. Procédé selon la revendication 8 **caractérisé par le fait que** l'anhydride carboxylique est l'anhydride acétique.

12. Procédé selon l'une des revendications 1 à 11 **caractérisé par le fait que** la quantité de base exprimée par le rapport entre le nombre de moles de base et le nombre de moles de substrat de départ répondant préférentiellement à la formule (I) varie entre 0,05 et 1,0 et se situe de préférence, entre 0,1 et 0,2.

13. Procédé selon l'une des revendications 1 à 10 **caractérisé par le fait que** la quantité d'anhydride d'acide carboxylique mise en oeuvre est telle que le rapport molaire anhydride d'acide carboxylique/composé de formule (I) varie entre 2 et 10.

14. Procédé selon la revendication 1 **caractérisé par le fait qu'**il consiste à effectuer la cyclisation du substrat de départ en l'absence ou en présence d'un solvant organique.

15. Procédé selon la revendication 14 **caractérisé par** le fait le solvant organique est choisi parmi : les hydrocarbures aromatiques, halogénés ou non, les éther-oxydes aliphatiques, cycloaliphatiques ou aromatiques les carboxamides linéaires ou cycliques . le diméthylsulfoxyde (DMSO) ; l'hexaméthylphosphotriamide (HMPT) ; la tétraméthylurée ; les composés nitrés ; les nitriles aliphatiques ou aromatiques ; la tétraméthylène sulfone (sulfolane) ; et les mélanges des différents solvants précités.

16. Procédé selon la revendication 14 **caractérisé par le fait que** le solvant organique est le diméthylformamide.

17. Procédé selon la revendication 14 **caractérisé par le fait que** la quantité d'anhydride d'acide carboxylique mise en oeuvre est telle que le rapport molaire anhydride d'acide carboxylique/composé de formule (I) varie entre 1 et 3.

18. Procédé selon la revendication 14 **caractérisé par le fait que** la quantité de solvant organique à mettre en oeuvre est déterminée de telle sorte que la concentration du substrat dans le solvant organique soit de préférence comprise entre 1 et 5 mol/litre et plus préférentiellement entre 2 et 3 mol/litre.

19. Procédé selon l'une des revendications 1 à 18 **caractérisé par le fait que** la réaction de cyclisation a lieu à une température qui est située entre 50°C et 160°C, de préférence, entre 100°C et 140°C.

20. Procédé selon l'une des revendications 1 à 19 **caractérisé par le fait que** le produit cyclisé est récupéré dans la phase organique.

21. Procédé selon l'une des revendications 1 à 20 **caractérisé par le fait que** l'on effectue avant la cyclisation, la saponification du composé de formule (I') lorsqu'il comprend à titre de groupe COOR₂, une fonction ester au lieu d'une fonction carboxylique COOH.

22. Procédé selon l'une des revendications 1 à 20 **caractérisé par le fait que** le produit cyclisé répond à la formule (V) suivante : 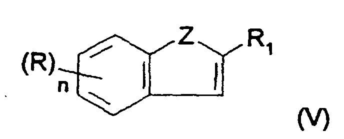 dans ladite formule (V), R, R₁, Z et n ont la signification donnée précédemment dans l'une des revendications 2 à 5.

23. Procédé selon l'une des revendications 1 à 20 **caractérisé par le fait que** le produit cyclisé répond à la formule (V') suivante : 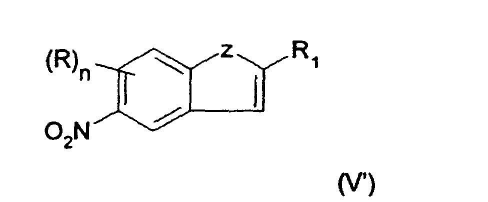 dans ladite formule (V'), R, R₁, Z et n ont la signification donnée précédemment dans l'une des revendications 2 à 5.

24. Procédé selon la revendication 23 **caractérisé par le fait que** le produit cyclisé est le 2-n-butyl-5-nitrobenzofurane.

## Claims

1. A process for preparing a benzofuran or benzothiophene type compound from an aromatic compound comprising a benzene ring carrying firstly, a side chain comprising at least two carbon atoms, one of the carbon atoms being connected to the benzene ring via an oxygen or sulphur atom, the other carbon atom being in the carboxylic form, and secondly, carrying a formyl group in the position ortho to said chain, **characterized in that** it consists of cyclizing the latter in a medium comprising a carboxylic acid anhydride and in the presence of a base selected from metal or ammonium carbonates and/or bicarbonates.

2. A process according to claim 1, **characterized in that** the aromatic starting compound has the following formula: 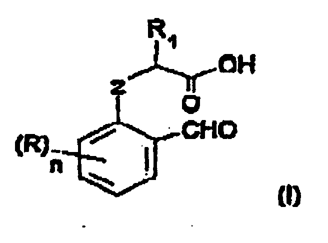 in which formula (I):
• R₁ represents a hydrogen atom, a linear or branched alkyl group containing 1 to 12 carbon atoms, or a phenyl group that is optionally substituted with an alkyl group containing 1 to 4 carbon atoms or a halogenophenyl group;
• Z represents an oxygen or sulphur atom;
• R represents a hydrogen atom or a substituent;
• n is a number equal to 0. 1, 2 or 3, preferably 0;
• when n is greater than 1, two groups R and the two successive atoms on the benzene ring may between them form a saturated, unsaturated or aromatic cycle containing 5 to 7 carbon atoms.

3. A process according to claim 2, **characterized in that** R represents a hydrogen atom or one of the following groups:
• a nitro group;
• a hydroxyl group;
• a linear or branched alkyl group containing 1 to 6 carbon atoms, preferably 1 to 4 carbon atoms, such as methyl, ethyl, propyl, isopropyl, butyl, isobutyl, sec-butyl or tert-butyl;
• an alkoxy group containing 1 to 6 carbon atoms, preferably 1 to 4 carbon atoms;
• an alkylamide group containing 1 to 6 carbon atoms, preferably 1 to 4 carbon atoms;
• a carboxamide group;
• a halogen atom;
• a trifluoromethyl group.

4. A process according to claim 1, **characterized in that** the aromatic starting compound has the following formula: 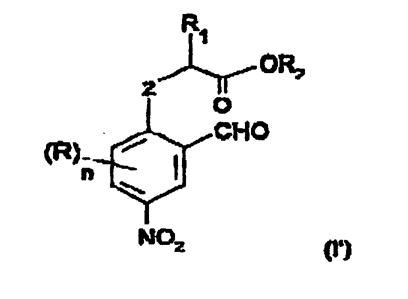 in which:
• R₁ represents a linear or branched alkyl group containing 1 to 12 carbon atoms, or a phenyl group that is optionally substituted with an alkyl group containing 1 to 4 carbon atoms or a halogenophenyl group;
• R₂ represents a hydrogen atom, a hydrocarbon group containing 1 to 12 carbon atoms which may be a linear or branched alkyl group, a cycloalkyl group, a phenyl group or a phenylalkyl group;
• Z represents an oxygen or sulphur atom;
• R represents a hydrogen atom or a substituent;
• n is a number equal to 0. 1, 2 or 3, preferably 0;
• when n is greater than 1, two groups R and the two successive atoms on the benzene ring may between them form a saturated, unsaturated or aromatic cycle containing 5 to 7 carbon atoms.

5. A process according to claim 2 or claim 4, **characterized in that** R represents a hydrogen atom, a methyl or ethyl group, or a methoxy or ethoxy group.

6. A process according to claim 2 or claim 4, **characterized in that** R₁ represents an alkyl group containing 1 to 4 carbon atoms.

7. A process according to claim 1, **characterized in that** the base used is sodium carbonate or potassium carbonate.

8. A process according to claim 1, **characterized in that** the carboxylic acid anhydride has the following formula: 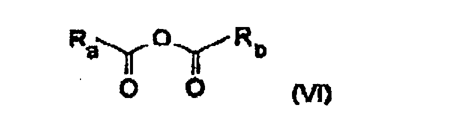 in which formula (VI):
• Rₐ and R_{b}, which may be identical or different, represent a monovalent hydrocarbon group that may or may not be substituted, which can be a linear or branched, saturated or unsaturated acyclic aliphatic group; or a monocyclic saturated, unsaturated or aromatic carbocyclic group;
• Rₐ and R_{b} can together form a divalent linear or branched, saturated or unsaturated aliphatic group containing at least 2 carbon atoms.

9. A process according to claim 8, **characterized in that** the carboxylic anhydride is a cyclic anhydride containing 5 to 10 carbon atoms in the cycle that may or may not contain a double bond, one of the atoms possibly being replaced by an oxygen atom.

10. A process according to claim 8, **characterized in that** the carboxylic anhydride is a non cyclic anhydride with formula (VI), in which groups Rₐ and R_{b}, which may be identical or different, represent:
• a linear or branched acyclic aliphatic group preferably containing 1 to 24, more preferably 1 to 12 carbon atoms, which may be saturated or comprise one or more unsaturated bonds in its chain, generally 1 to 3 unsaturated bonds, which may be simple double bonds: the hydrocarbon chain may be interrupted by one of the following groups: -O-; -CO-; and/or carry one or more substituents, in particular: -X; -CX₃;
• a saturated, unsaturated or aromatic carbocyclic group containing 3 to 8 carbon atoms, preferably 6 carbon atoms, optionally carrying one or more halogen atoms, preferably chlorine or bromine.

11. A process according to claim 8, **characterized in that** the carboxylic anhydride is acetic anhydride.

12. A process according to one of claims 1 to 11, **characterized in that** the quantity of base, expressed as the ratio between the number of moles of base and the number of moles of starting substrate preferably satisfying formula (I) is in the range 0.05 to 1.0, preferably in the range 0.1 to 0.2.

13. A process according to one of claims 1 to 10, **characterized in that** the quantity of carboxylic acid anhydride employed is such that the mole ratio of the carboxylic acid anhydride/compound with formula (I) is in the range 2 to 10.

14. A process according to claim 1, **characterized in that** it consists of cyclizing the starting substrate in the absence or presence of an organic solvent.

15. A process according to claim 14, **characterized in that** the organic solvent is selected from: aromatic hydrocarbons, which may or may not be halogenated, aliphatic, cycloaliphatic or aromatic ether-oxides, linear or cyclic carboxamides, dimethylsulphoxide (DMSO); hexamethylphosphotriamide (HMPT); tetramethylurea; nitro compounds; aliphatic or aromatic nitriles; tetramethylene sulphone (sulpholane); and mixtures of the different said solvents.

16. A process according to claim 14, **characterized in that** the organic solvent is dimethylformamide.

17. A process according to claim 14, **characterized in that** the quantity of carboxylic acid anhydride employed is such that the mole ratio of the carboxylic acid anhydride/compound with formula (I) is in the range 1 to 3.

18. A process according to claim 14, **characterized in that** the quantity of organic solvent used is determined such that the concentration of substrate in the organic solvent is preferably in the range 1 to 5 mole/litre, more preferably in the range 2 to 3 mole/litre.

19. A process according to any one of claims 1 to 18, **characterized in that** the cyclization reaction takes place at a temperature in the range 50°C to 160°C, preferably in the range 100°C to 140°C.

20. A process according to any one of claims 1 to 19, **characterized in that** the cyclized product is recovered in the organic phase.

21. A process according to one of claims 1 to 20, **characterized in that** prior to cyclizing, the compound with formula (I') is saponified when it contains an ester function instead of a carboxylic function COOH as the group COOR₂.

22. A process according to one of claims 1 to 20, **characterized in that** the cyclized product has the following formula (V): 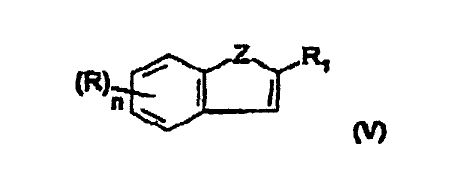 in which formula (V), R, R₁, Z and n have the meanings given above in any one of claims 2 to 5.

23. A process according to one of claims 1 to 20, **characterized in that** the cyclized product has the following formula (V'): 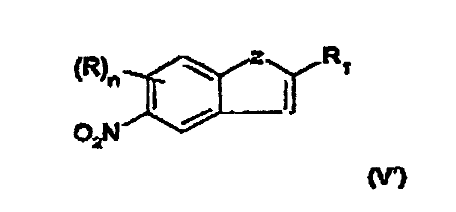 in which formula (V'), R, R₁, Z and n have the meanings given above in any one of claims 2 to 5.

24. A process according to claim23, **characterized in that** the cyclized product is 2-n-butyl-5-nitrobenzofuran.

## Patentansprüche

1. Verfahren zur Herstellung einer Verbindung vom Typ Benzofuran oder Benzothiophen ausgehend von einer aromatischen Verbindung mit einem Benzolring, welcher einerseits eine Seitenkette mit mindestens zwei Kohlenstoffatomen, wobei eines der Kohlenstoffatome mit dem Benzolring über ein Sauerstoff- oder Schwefelatom verbunden ist und das andere Kohlenstoffatom in carboxylischer Form vorliegt, und andererseits eine Formylgruppe in ortho-Position in bezug auf diese Kette trägt, **dadurch gekennzeichnet, daß** man den Ringschluß (Cyclisierung) des letzteren in einem Milieu, welches ein Carbonsäureanhydrid umfaßt, und in Gegenwart einer Base, die ausgewählt ist aus Metall- oder Ammoniumcarbonaten und/oder -hydrogencarbonaten, durchführt.

2. Verfahren nach Anspruch 1, **dadurch gekennzeichnet, daß** die aromatische Ausgangsverbindung der folgenden Formel entspricht: 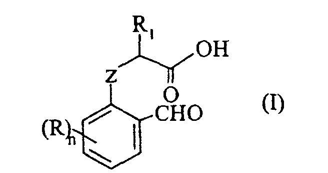 wobei in der Formel (I):
- R₁ ein Wasserstoffatom, eine lineare oder verzweigte Alkylgruppe mit 1 bis 12 Kohlenstoffatomen, eine gegebenenfalls mit einer Alkylgruppe mit 1 bis 4 Kohlenstoffatem substituierte Phenylgruppe oder eine Halogenphenylgruppe darstellt,
- Z ein Sauerstoff- oder ein Schwefelatom darstellt,
- R ein Wasserstoffatom oder einen Substituenten darstellt,
- n eine ganze Zahl 0, 1, 2 oder 3, vorzugsweise 0, darstellt,
- wobei, wenn n größer als 1 ist, zwei Gruppen R und die 2 aufeinanderfolgenden Atome des Benzolrings untereinander einen gesättigten, ungesättigten oder aromatischen Ring mit 5 bis 7 Kohlenstoffatomen bilden können.

3. Verfahren nach Anspruch 2, **dadurch gekennzeichnet, daß** R ein Wasserstoffatom oder eine der folgenden Gruppen darstellt:
- eine Nitrogruppe,
- eine Hydroxylgruppe,
- eine lineare oder verzweigte Alkylgruppe mit 1 bis 6 Kohlenstoffatomen, vorzugsweise 1 bis 4 Kohlenstoffatomen, wie Methyl, Ethyl, Propyl, Isopropyl, Butyl, Isobutyl, sek.-Butyl und tert.-Butyl,
- eine Alkoxygruppe mit 1 bis 6 Kohlenstoffatomen, vorzugsweise 1 bis 4 Kohlenstoffatomen,
- eine Estergruppe mit 1 bis 10 Kohlenstoffatomen, vorzugsweise 1 bis 4 Kohlenstoffatomen,
- eine Alkylamidgruppe mit 1 bis 6 Kohlenstoffatomen, vorzugsweise 1 bis 4 Kohlenstoffatomen,
- eine Carboxamidgruppe,
- ein Halogenatom,
- eine Trifluormethylgruppe.

4. Verfahren nach Anspruch 1, **dadurch gekennzeichnet, daß** die aromatische Ausgangsverbindung der folgenden Formel entspricht: 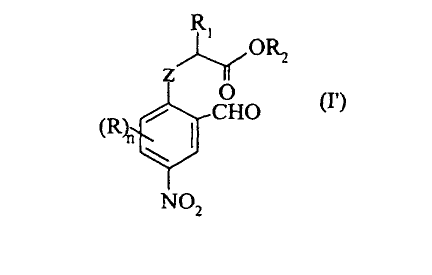 wobei:
- R₁ eine lineare oder verzweigte Alkylgruppe mit 1 bis 12 Kohlenstoffatomen, eine gegebenenfalls mit einer Alkylgruppe mit 1 bis 4 Kohlenstoffatomen substituierte Phenylgruppe oder ein Halogenphenylgruppe darstellt,
- R₂ ein Wasserstoffatom, eine Kohlenwasserstoffgruppe mit 1 bis 12 Kohlenstoffatomen, die eine lineare oder verzweigte Alkylgruppe sein kann, eine Cycloalkylgruppe, eine Phenylgruppe oder eine Phenylalkylgruppe darstellt,
- Z ein Wasserstoff- oder ein Schwefelatom darstellt,
- R ein Wasserstoffatom oder einen Substituenten darstellt,
- n eine ganze Zahl 0, 1, 2 oder 3, vorzugsweise 0, darstellt,
- wobei, wenn n größer als 1 ist, zwei Gruppen R und die 2 aufeinanderfolgenden Atome des Benzolrings untereinander einen gesättigten, ungesättigten oder aromatischen Ring mit 5 bis 7 Kohlenstoffatomen bilden können.

5. Verfahren nach einem der Ansprüche 2 und 4, **dadurch gekennzeichnet, daß** R ein Wasserstoffatom, eine Methyl- oder Ethylgruppe oder eine Methoxyoder Ethoxygruppe darstellt.

6. Verfahren nach einem der Ansprüche 2 und 4, **dadurch gekennzeichnet, daß** R₁ eine Alkylgruppe mit 1 bis 4 Kohlenstoffatomen darstellt.

7. Verfahren nach Anspruch 1, **dadurch gekennzeichnet, daß** die eingesetzte Base Natriumcarbonat oder Kaliumcarbonat ist.

8. Verfahren nach Anspruch 1, **dadurch gekennzeichnet, daß** das Carbonsäureanhydrid der folgenden Formel entspricht: 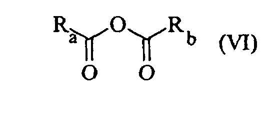 wobei in der Formel (VI):
- Rₐ und R_{b}, identisch oder verschieden, eine einwertige (monovalente) Kohlenwasserstoffgruppe, die gegebenenfalls substituiert ist und eine lineare oder verzweigte, gesättigte oder ungesättigte, acyclische aliphatische Gruppe oder eine monocyclische, gesättigte, ungesättigte oder aromatische carbocyclische Gruppe sein kann,
- wobei Rₐ und R_{b} gemeinsam eine lineare oder verzweigte, gesättigte oder ungesättigte, aliphatische zweiwertige (divalente) Gruppe mit mindestens zwei Kohlenstoffatomen bilden können.

9. Verfahren nach Anspruch 8, **dadurch gekennzeichnet, daß** das Carbonsäureanhydrid ein ringförmiges Anhydrid mit 5 bis 10 Kohlenstoffatomen im Ring ist, der gegebenenfalls eine Doppelbindung enthalten kann, wobei eines der Atome durch ein Sauerstoffatom ersetzt sein kann.

10. Verfahren nach Anspruch 8, **dadurch gekennzeichnet, daß** das Carbonsäureanhydrid ein nichtringförmiges Anhydrid der allgemeinen Formel (VI) ist, in der die Gruppen Rₐ und R_{b}, identisch oder verschieden, darstellen:
- eine lineare oder verzweigte, acyclische aliphatische Gruppe mit vorzugsweise 1 bis 24, bevorzugt 1 bis 12 Kohlenstoffatomen, die gesättigt ist oder ein oder mehrere ungesättigte Bindungen in der Kette, im allgemeinen 1 bis 3 ungesättigte Bindungen, umfaßt, bei denen es sich um einfache Doppelbindungen handeln kann, wobei die Kohlenwasserstoffkette unterbrochen sein kann durch eine der folgenden Gruppen: -O-; -CO-; und/oder Träger eines oder mehrerer Substituenten, insbesondere -X; -CX₃, sein kann,
- eine gesättigte, ungesättigte oder aromatische carbocyclische Gruppe mit 3 bis 8 Kohlenstoffatomen, vorzugsweise 6 Kohlenstoffatomen, die gegebenenfalls Träger eines oder mehrerer Halogenatome, vorzugsweise Chlor oder Brom, ist.

11. Verfahren nach Anspruch 8, **dadurch gekennzeichnet, daß** das Carbonsäureanhydrid Essigsäureanhydrid ist.

12. Verfahren nach einem der Ansprüche 1 bis 11, **dadurch gekennzeichnet, daß** die Menge an Base, berechnet als Verhältnis zwischen der Molanzahl an Base und der Molanzahl an Ausgangssubstrat (Edukt), das vorzugsweise der Formel (I) entspricht, zwischen 0,05 und 1,0 variiert und vorzugsweise zwischen 0,1 und 0,2 liegt.

13. Verfahren nach einem der Ansprüche 1 bis 10, **dadurch gekennzeichnet, daß** die eingesetzte Menge an Carbonsäureanhydrid derart ist, daß das Molverhältnis von Carbonsäureanhydrid/Verbindung der Formel (I) zwischen 2 und 10 variiert.

14. Verfahren nach Anspruch 1, **dadurch gekennzeichnet, daß** man den Ringschluß (Cyclisierung) des Ausgangssubstrats (Edukts) in Abwesenheit oder in Anwesenheit eines organischen Lösemittels durchführt.

15. Verfahren nach Anspruch 14, **dadurch gekennzeichnet, daß** das organische Lösemittel ausgewählt ist aus gegebenenfalls halogenierten aromatischen Kohlenwasserstoffen, aliphatischen, cycloaliphatischen oder aromatischen Etheroxiden, linearen oder ringförmigen Carboxamiden, Dimethylsulfoxid (DMSO), Hexamethylphosphotriamid (HMPT), Tetramethylharnstoff, nitrierten Verbindungen, aliphatischen oder aromatischen Nitrilen, Tetramethylensulfon (Sulfolan) und Mischungen der verschiedenen vorgenannten Lösemitteln.

16. Verfahren nach Anspruch 14, **dadurch gekennzeichnet, daß** das organische Lösemittel Dimethylformamid ist.

17. Verfahren nach Anspruch 14, **dadurch gekennzeichnet, daß** die eingesetzte Menge an Carbonsäureanhydrid derart ist, daß das Molverhältnis Carbonsäureanhydrid/Verbindung der Formel (I) zwischen 1 und 3 variiert.

18. Verfahren nach Anspruch 14, **dadurch gekennzeichnet, daß** die einzusetzende Menge an organischem Lösemittel derart bestimmt wird, daß die Konzentration des Substrats (Edukts) in dem organischen Lösemittel vorzugsweise zwischen 1 und 5 mol/l, bevorzugt zwischen 2 und 3 mol/l, liegt.

19. Verfahren nach einem der Ansprüche 1 bis 18, **dadurch gekennzeichnet, daß** die Ringschlußreaktion (Cyclisierungsreaktion) bei einer Temperatur stattfindet, die zwischen 50 °C und 160 °C, vorzugsweise zwischen 100 °C und 140 °C, liegt.

20. Verfahren nach einem der Ansprüche 1 bis 19, **dadurch gekennzeichnet, daß** das Ringschlußprodukt (Cyclisierungsprodukt) in der organischen Phase erhalten wird.

21. Verfahren nach einem der Ansprüche 1 bis 20, **dadurch gekennzeichnet, daß** man vor dem Ringschluß (Cyclisierung) eine Verseifung der Verbindung der Formel (I') durchführt, wenn sie als Gruppe COOR₂ eine Esterfunktion anstelle einer Carbonsäurefunktion COOH umfaßt.

22. Verfahren nach einem der Ansprüche 1 bis 20, **dadurch gekennzeichnet, daß** das Ringschlußprodukt (Cyclisierungsprodukt) der folgenden Formel (V) entspricht: 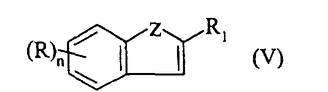 wobei in der Formel (V) R, R₁, Z und n die zuvor in den Ansprüchen 2 bis 5 angegebene Bedeutung haben.

23. Verfahren nach einem der Ansprüche 1 bis 20, **dadurch gekennzeichnet, daß** das Ringschlußprodukt (Cyclisierungsprodukt) der folgenden Formel (V') entspricht: 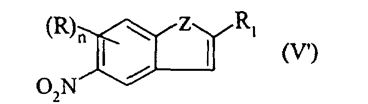 wobei in der Formel (V') R, R₁, Z und n die zuvor in den Ansprüchen 2 bis 5 angegebene Bedeutung haben.

24. Verfahren nach Anspruch 23, **dadurch gekennzeichnet, daß** das Ringschlußprodukt (Cyclisierungsprodukt) 2-n-Butyl-5-nitrobenzofuran ist.
